# EUROPEAN PATENT SPECIFICATION

(11) **EP 3 270 873 B1**
(45) Date of publication and mention of the grant of the patent: **25.11.2020**
(21) Application number: 16721030.1
(22) Date of filing: 21.04.2016
(51) Int. Cl.: A61Q 11/00, A61K 8/24, A61K 8/34, A61K 8/86, A61K 8/19

(54) **CALCIUM-BASED DENTIFRICES FOR ENHANCED UPTAKE OF ACTIVE INGREDIENTS**
AUF CALCIUM BASIERENDE ZAHNPASTEN ZUR VERSTÄRKTEN AUFNAHME VON WIRKSTOFFEN
DENTIFRICES À BASE DE CALCIUM POUR UNE MEILLEURE ABSORPTION DE PRINCIPES ACTIFS

(30) Priority: 23.04.2015 IN 1132DE2015
(43) Date of publication of application: 24.01.2018
(73) Proprietor: Colgate-Palmolive Company, New York, NY 10022 (US)
(72) Inventor: POTNIS, Shashank Vishwanath, Thane (W) 400610 (IN); PLATA, Rolando, Picataqway, NJ 08854 (US); SIRDESAI, Amit, Mumbai 400080 (IN); TEMBE, Sagar, Mumbai 400068 (IN); LEWIS, Navin, Mumbai 400031 (IN)
(74) Representative: Wibbelmann, Jobst
(86) International application number: PCT/US2016/028629
(87) International publication number: WO 2016/172334

(56) References cited:
- EP-A2- 0 267 788
- WO-A1-2011/055707
- US-A- 3 629 398
- US-A- 3 885 028
- US-A- 5 032 385
- US-A- 6 113 884
- US-A1- 2013 029 294

## Description

### BACKGROUND

Oral care compositions such as toothpastes are generally of two types, opaque and transparent. Opaque toothpastes typically contain a calcium-based abrasive, such as calcium carbonate. Transparent (e.g., gel) toothpastes, in contrast, usually contain silica as the abrasive. Either of these formulations may beneficially include an anti-bacterial agent such as 5-chloro-2-(2,4-dichlorophenoxy)phenol (triclosan). However, delivery of anti-bacterial agents typically requires the use of vinylmethyl ether and maleic acid polymers, *e.g.* GANTREZ™. Such polymers, which are widely used in silica based toothpastes, improve the uptake and retention of anti-bacterial agents by, for example, trapping and then adhering the anti-bacterial agent to the teeth and gums. However, the use of such polymers is limited in calcium-based dentifrices since they may cause chelation and precipitation of calcium ions as described, for example, in WO2013/007571. Further, vinylmethyl ether and maleic acid polymer delivery vehicles may be costly, thus reducing the commercial viability of calcium-based dentifrices, which are mass market products.

Calcium-based abrasive formulations using reduced amounts of GANTREZ™ that may be effective for triclosan delivery are known. These formulations are reported to contain about 0.75 wt% GANTREZ™ in combination with a smectite clay, such as VEGGUM® HV (usually about 0.8 wt%), viscosity builders, such as carboxymethylcellulose, thickener silica, such as, MFIL®-P and alkalizers, such as sodium silicate (1.75 wt%), which act to neutralize GANTREZ™.

The inclusion of even reduced amounts of GANTREZ™, however, in combination with VEGGUM® HV clay may prohibitively increase formulation costs. Further, formulations containing clays are known to increase batch cycle times due to mixing and hydration requirements. Accordingly, there is a desire in the art to improve the uptake of active ingredients such as triclosan in calcium-based oral care compositions without the use of GANTREZ™ or similar polymers, and further without the use of large amounts of viscosity modifiers and/or sodium silicate to attain cost effective calcium-based oral care formulations which may be quickly and easily produced.
US 5 032 385 A discloses a toothpaste formulation comprising chalk, triclosan and polyethylene glycol 300.

### BRIEF SUMMARY

The present invention concerns an oral care composition: (i) a calcium-based abrasive; (ii) a polyethylene glycol having an average molecular weight ranging from 1500 to 6000 daltons in an amount of 0.1 wt% to 0.3 wt%; (iii) an anti-bacterial agent; and (iv) an orally acceptable vehicle, wherin the antibacterial agent is a halogenated diphenyl ether.

Also provided herein is a method of preparing said oral care composition including: combining a humectant and water to form an orally acceptable vehicle; b) adding a calcium-based abrasive to the orally acceptable vehicle: c) combining a polyethylene glycol having an average molecular weight ranging from about 1,500 to about 6,000 daltons, an anti-bacterial agent, and, optionally, an ingredient selected from the group consisting of a flavoring agent and a preservative to form a premix; d) adding the premix of step c) to the orally acceptable vehicle containing the calcium base abrasive of step b) to form the oral care composition.

The present disclosure also provides a method of promoting oral health, the method including contacting a mouth surface with an oral care composition including: (i) a calcium-based abrasive; (ii) a polyethylene glycol having an average molecular weight ranging from about 1500 to about 6000 daltons; (iii) an anti-bacterial agent and (iv) an orally acceptable vehicle.

Further areas of applicability of the present disclosure will become apparent from the detailed description provided hereinafter. It should be understood that the detailed description and specific examples, while indicating typical embodiments, are intended for purposes of illustration only and are not intended to limit the scope of the disclosure.

### BRIEF DESCRIPTION OF THE DRAWINGS

The present disclosure will become more fully understood from the detailed description and the accompanying drawings, wherein:
FIG. 1 is a bar graph that shows an increase in uptake and retention of triclosan in calcium-based dentifrice formulations containing polyethylene glycol having an average molecular weight of 6,000 daltons (PEG 6000) at a reduced pH as described in the Examples.
FIG. 2 is a bar graph that shows the effect of sodium silicate on uptake and retention of triclosan in calcium-based dentifrice formulations as described in the Examples.

### DETAILED DESCRIPTION

The following description of the embodiments is merely exemplary in nature and is in no way intended to limit the disclosure, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

### Compositions

The present inventors surprisingly recognized that increased retention and uptake of an anti-bacterial agent such as triclosan may be obtained in calcium-based oral care compositions containing a high molecular weight polyethylene glycol such as PEG 6000. The present inventors further surprisingly recognized that improved uptake and retention of anti-bacterial agents, such as triclosan, can also be obtained at pH values, *e.g.,* less than 9.0. Moreover, the present calcium-based oral care compositions advantageously can be prepared without the need for large amounts of viscosity modifiers, less or no sodium silicate and within a shortened batch cycle time in comparison to prior art formulations.

An "oral care composition" as used herein refers to a composition, which is retained in the oral cavity for a time sufficient to contact substantially all of the dental surfaces and/or oral tissues for purposes of oral activity. The oral care composition of the present disclosure may be in various forms including mouth rinses or dentifrices. Dentifrices may include a dental tablet, toothpaste (dental cream), tooth powders, gel, liquid, liquigel, or any other form known to one of skill in the art.

### The calcium-based abrasive

In some embodiments, the oral care compositions of the present disclosure include a calcium-based dental abrasive or a combination of calcium-based dental abrasives. As used herein, the term "abrasive" or "abrasive agent" includes materials commonly referred to as "polishing agents." Any orally acceptable calcium-based abrasive can be used, but typically, type, fineness (particle size) and amount of abrasive should be selected so that tooth enamel is not excessively abraded in normal use of the composition. Suitable calcium-based abrasive polishing agents include precipitated calcium carbonate (PCC), chalk, dicalcium phosphate and its dihydrate forms, calcium pyrophosphate, tricalcium phosphate, dicalcium orthophosphate, calcium pyrophosphate, n-calcium pyrophosphate, Natural calcium carbonate (NCC) and the like. Typically, PCC is used.

Typically, the oral care compositions of the present disclosure include only calcium based abrasives. However, oral care compositions including silica based abrasives are also contemplated. Formulations including only silica abrasives may also be beneficially used in combination with the described polyethylene glycols at a suitable pH as described herein for enhancing anti-bacterial uptake and retention. Suitable silica based abrasives include, without limitation, silica in the form of silica gel, hydrated silica or precipitated silica.

Other contemplated embodiments include a mixture of calcium based abrasives and silica based abrasives. Such mixtures may also be combined with the high molecular weight polyethylene glycols, anti-bacterial agents at a suitable pH as described herein for enhancing the uptake and retention of anti-bacterial agents.

In yet other contemplated embodiments, the oral care compositions include multiphase dentifrices. Such multiphase dentifrices include, for example, an opaque phase including a calcium based abrasive, and a gel phase including silica abrasives. The opaque phase, gel phase or both phases may include one or more anti-bacterial agents and a polyethylene glycol as described herein, such as PEG-6000 at a suitable pH, to enhance uptake and retention of an anti-bacterial agent, such as triclosan.

In some embodiments, the average particle size of the abrasive is generally about 0.1 to about 30 µm, for example about 1 to about 20 µm or about 5 to about 15 µm.

In some embodiments, one or more calcium-based and/or silica based abrasives is included in the oral care formulations of the present disclosure in an amount ranging from about 5 to about 75 wt%, such as about 30 to about 60 wt%, such as about 35 to about 55 wt%.

### Polyethylene Glycol

In some embodiments, the oral care compositions of the present disclosure include polyethylene glycol (PEG) having a molecular weight ranging from 1,500 to 6,000 daltons, such as about 4,000 to 6,000 daltons, such as about 5,000 to 6,000 daltons, more typically 6,000 daltons.

The amount of polyethylene glycol as described herein for use with the present oral care compositions ranges from 0.1 wt% to 0.3 wt%. Typically, 0.3 wt% of polyethylene glycol, such as PEG 6000 is used.

In some embodiments, the polyethylene glycols having a molecular weight ranging from 1,500 to 6,000 daltons that are used with the present disclosure are in solid form at 20°C, *e.g.* flakes, powders, or waxes. Suitable commercially available polyethylene glycol polymers such as PEG 6000 may be obtained from Clariant, India and BASF, USA. Other commercially available sources may be obtained from Sigma-Aldrich, St. Louis, MO, USA, Santa Cruz Biotechnology, Inc., Dallas Texas, USA and Hallstar, Chicago, Il, USA, for example.

Without being bound by theory, it is believed that the polyethylene glycols described herein, *e.g.* PEG-6000, attaches to tooth surfaces to form a continuous film over the surfaces, thereby preventing bacterial attachment to tooth surfaces. It is further contemplated that an anti-bacterial agent as disclosed herein forms a complex or other form of association with the polyethylene glycol, thus forming a film of a complex or the like over tooth surfaces. The delivery and retention of the anti-bacterial agents and the film-forming properties of the polyethylene glycols described herein appear to make tooth surfaces unfavorable for bacterial accumulation, particularly since the direct bacteriostatic action of the anti-bacterial agent complexed with the polyethylene glycol controls bacterial growth. Therefore, through the combination of 1) enhanced delivery, 2) long retention time on tooth surfaces and 3) prevention of bacterial attachment to tooth surfaces, the oral care composition of the present disclosure is made efficacious for reducing plaque. Moreover, this reduction in plaque is achieved without causing chelation or precipitation of calcium. Accordingly, the polyethylene glycols of the present disclosure may be used in combination with the present oral care compositions comprising calcium-based abrasives.

In some embodiments, the oral care compositions of the present disclosure do not include copolymers of monoalkyl esters of poly (methyl vinyl ether/maleic acid) with varying ester groups such as butyl ester of polyvinyl methyl ether/maleic acid (PVM/MA) copolymer, isopropyl ester of PVM/MA copolymer and ethyl ester of PVM/MA copolymer, *e.g*. the GANTREZ™ series of polymers, which are commercially available, for example, from Ashland Inc. Covington, KY.

### Anti-bacterial agents

The present oral compositions include anti-bacterial agents. The anti-bacterial agents, which may be delivered and retained via the polyethylene glycols described herein include halogenated diphenyl ethers. The anti-bacterial agent present in the oral care composition of the present disclosure is in an effective antiplaque amount of about 0.5 wt% to about 1.0 wt%, such as about 0.25 wt% to about 0.35 wt% by weight, typically about 0.3 wt%.

Examples of halogenated diphenyl ethers used with the present calcium-based oral care compositions include but are not limited to 2',4,4'-trichloro-2-hydroxy-diphenyl ether (triclosan), 2,2'-dihydroxy-5,5'-dibromo-diphenyl ether, halogenated salicylanilides such as 4'5-dibromosalicylanilide, 3,4',5-trichlorosalcylanilide, 3,4',5-tribromosalicylanilide, 2,3,3',5-tetrachlorosalicylanilide, 3,3',5-tetrachlorosalicylanilide, 3,5-dibromo-3'-trifluoromethyl salicylanilide, 5-n-octanoyl-3'-trifluoromethyl salicylanilide, 3,5-dibromo-4'-trifluoromethyl salicylanilide, 3,5-dibromo-3'-trifluoro methyl salicylanilide (fluorophene), benzoic esters, methyl-p-hydroxybenzoic ester, ethyl-p-hydroxybenzoic ester, propyl-p-hydroxybenzoic ester, butyl-p-hydroxybenzoic ester, halogenated carbanilides, 3,4,4'-trichlorocarbanilide, 3-trifluoromethyl-4,4'-dichlorocarbanilide, 3,3',4-trichlorocarbanilide or combinations thereof.

In some embodiments, the present oral care compositions may further include phenolic compounds and homologs of phenolic compounds. Examples include 2-methoxy-4-(2-propenyl)-phenol (eugenol), 2-isopropyl-5-methyl-phenol (thymol), mono- and poly-alkyl and aralkyl halophenols, methyl-p-chlorophenol, ethyl-p-chlorophenol, n-propyl-p-chlorophenol, n-butyl-p-chlorophenol, n-amyl-p-chlorophenol, sec-amyl-p-chlorophenol, n-hexyl-p-chlorophenol, cyclohexyl-p-chlorophenol, n-heptyl-p-chlorophenol, n-octyl-p-chlorophenol, 3,4,5,6-terabromo-2-methylphenol, 5-methyl-2-pentylphenol, 4-isopropyl-3-methylphenol and 5-chloro-2-hydroxydiphenylemthane.

Sesquiterpene alcohols such as farnesol, nerolidol, bisabolol, santalol and halogenated carbanilides such as 3,4,4'-trichlorocarbanilide 3-trifluoromethyl-4,4'-dichlorocarbanilide, 3,3,4'-trichlorocarbanilide are also contemplated. Other desirable anti-bacterial agents for use with the oral composition of the present disclosure are described in U.S. Patent No. 5,728, 756. Typically, triclosan as the halogenated diphenyl ether anti-bacterial agent is included in the oral care compositions of the present disclosure.

### pH

In some embodiments, the pH of the present oral care compositions may be adjusted with an acidic material or an alkaline material to achieve a particular pH value. In some embodiments, buffering agents are used to maintain a desired pH range of the present oral care compositions.

Examples of pH adjusting agents include citric acid, phosphoric acid, malic acid, pyrophosphoric acid, lactic acid, tartaric acid, glycerophosphoric acid, acetic acid, nitric acid, silicic acid, gluconic acid, maleic acid, aspartic acid, succinic acid, glucuronic acid, fumaric acid, adipic acid, glutamic acid, or chemically-acceptable salts thereof, such as sodium and potassium salts, hydrochloric acidsodium hydroxide, potassium hydroxide, *etc.* These pH adjusting agents can be used alone or in combination of two or more so as to adjust the pH of the present oral care composition within a range described above.

Suitable buffering agents, which may be used to maintain a desired pH of the present oral care compositions include, for example, soluble phosphate salts and sodium silicate. In some embodiments, sodium silicate is used as a buffering agent. The amount of buffering agent, typically sodium silicate, is usually within a range from about 0.01wt% to 5% wt%, such as 1% to 3% of the present oral care compositions.

In some embodiments, the pH of the oral care compositions is maintained in a range of from about 8.0 to about 10.5, such as from about 8.5 to about 9.4. More typically, the pH of the present compositions containing calcium-based abrasives is maintained in a range of less than about 9.0, such as less than about 8.9, such as less than about 8.7, such as less than about 8.6.

In some embodiments, the pH of the present oral care compositions is obtained by omitting buffering agents, such as by omitting sodium silicate from the present oral care compositions. Accordingly, in some embodiments, the present oral care compositions have a pH that is lower than art-known calcium-based dentifrices due to the absence of sodium silicate. For example, in some embodiments, the pH of the present oral care compositions containing calcium-based abrasives in the absence of a buffering agent such as sodium silicate is less than 9.0, such as less than 8.9, such as less than 8.7, such as less than 8.6.

### Optional Additional Active Ingredients

### Anti-caries agents

In some embodiments, the oral care composition of the present disclosure includes active ingredients in addition to calcium-based abrasives and anti-bacterial agents. For example, in some embodiments, the present oral care compositions include an orally acceptable source of fluoride ions, which serves as an anticaries agent. One or more such sources can be present. Suitable sources of fluoride ions include fluoride, monofluorophosphate and fluorosilicate salts as well as amine fluorides, including olaflur (N'-octadecyltrimethylendiamine-N,N,N'-tris(2-ethanol)-dihydrofluoride).

As an anticaries agent, one or more fluoride-releasing salts are optionally present in an amount providing a total of about 100 to about 20,000 ppm, about 200 to about 5,000 ppm, or about 500 to about 2,500 ppm, fluoride ions. Where sodium fluoride or monofluorophosphate is the sole fluoride-releasing salt present, illustratively an amount of about 0.01 wt% to about 5 wt%, about 0.05 wt% to about 1 wt% or about 0.1 wt% to about 0.8 wt%, sodium fluoride by weight can be present in the composition. Typically, sodium monofluorophosphate is used in the present oral care compositions in an amount ranging from about 0.7 to about 0.8 wt%, such as about 0.76 wt%.

### Bicarbonate

In some embodiments, the composition comprises at least one bicarbonate salt, useful, for example, to impart a "clean feel" to teeth and gums due to effervescence and release of carbon dioxide. Any orally acceptable bicarbonate can be used, including without limitation alkali metal bicarbonates such as sodium and potassium bicarbonates, ammonium bicarbonate and the like. One or more bicarbonate salts are optionally present in a total amount of about 0.1 wt% to about 50 wt%, for example about 0.5 wt% to about 5 wt% by weight of the composition.

### Anti-calculus agents

In some embodiments, further active ingredients are included in the calcium-based oral care compositions of the present disclosure. For example, anticalculus agents may be included. Suitable anti-calculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. In some embodiments, the anti-calculus agent is present in an amount of about 0.1 wt% to about 30 wt%. In some embodiments, the present oral care composition comprises a mixture of anti-calculus agents. In some embodiments, tetrasodium pyrophosphate (TSPP) and sodium tripolyphosphate (STPP) are used as the anti-calculus agents. In some embodiments, the anti-calculus agent comprises about 1 wt% to about 2 wt% TSPP, and about 0 wt% to about 7 wt% STPP.

Another optional component of the present oral care compositions is a synthetic anionic polymeric polycarboxylate (SAPP), which acts as a stabilizer for the polyphosphate anti-tartar agent and may help to block access of painful or pain-causing materials, such as sugars, to the tooth nerves.

### Whitening Agents

In some embodiments, the present oral care composition comprises a whitening agent. Any whitening agent known or developed in the art may be used in the present oral care compositions including peroxides, metal chlorites and persulfate. Exemplary peroxides include hydroperoxides, hydrogen peroxide, peroxides of alkali and alkaline earth metals, organic peroxy compounds, peroxy acids, pharmaceutically-acceptable salts thereof, and mixtures thereof.

Suitable peroxides of alkali and alkaline earth metals include lithium peroxide, potassium peroxide, sodium peroxide, magnesium peroxide, calcium peroxide, barium peroxide, and mixtures thereof. Organic peroxy compounds include urea peroxide, glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as perborate salts of alkali and alkaline earth metals such as lithium, potassium, sodium, magnesium, calcium and barium, and mixtures thereof. Typically, the whitening agent comprises hydrogen peroxide.

In some embodiments, the whitening agent comprises from about 0.1 wt% to about 50 wt%, such as from about 0.1 wt% to about 40% wt%, and more typically from about 0.1 wt% to about 30 wt%, of the present oral care compositions. In other embodiments, the oxidizing agent consists essentially of hydrogen peroxide. In some embodiments, the hydrogen peroxide comprises from about 0.1 wt% to about 3 wt%, such as from about 0.1 wt% to about 2 wt%, and more typically about 1 wt% to about 4 wt% of the present oral care compositions.

In some embodiments, a solid whitening agent, such as sodium perborate, urea peroxide, calcium chlorite, barium chlorite, magnesium chlorite, lithium chlorite, sodium chlorite, potassium chlorite or mixtures thereof are used in the present oral care compositions described herein.

In some embodiments, the whitening agent is bound, for example, to a polymer, such as PVP (poly(N-vinylpyrrolidone)). Suitable PVP complexes are disclosed, for example, in U.S. Patent No. 5,122,370.

### Stannous Ions

The present oral care compositions also may include a stannous ion or a stannous ion source to mitigate calcium loss. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01 wt% to about 10 wt%, for example about 0.1 wt% to about 7 wt% or about 1 wt% to about 5 wt%.

### Orally Acceptable Vehicle

The calcium-based abrasives, polyethylene glycol, anti-bacterial agent, such as triclosan, and any further desirable active agents or other oral care ingredients as described herein may be combined or associated with an orally acceptable vehicle. As used herein, an "orally acceptable vehicle" refers to a material or combination of materials that are safe for use in the compositions of the present disclosure, commensurate with a reasonable benefit/risk ratio, with which any desired active ingredients may be associated while retaining significant efficacy.

In some embodiments, the orally acceptable vehicle is a low water content orally acceptable vehicle and may include any known ingredients or additives. For example, the orally acceptable vehicle may include liquid mixtures of water and humectants, such as glycerin and/or sorbitol. In other embodiments, the orally acceptable vehicle may include liquid mixtures of water and sorbitol.

Water employed in the preparation of suitable oral care compositions of the present disclosure should preferably be deionized and free of organic impurities. In some embodiments, the water content of the present oral compositions is less than about 20 wt%, less than about 10 wt%, less than about 8 wt% less than about 4 wt%, less than about 2 wt%, less than about 1 wt% or less than about 0.1 wt%. Typically, the water content of the present oral care compositions is less than about 17%, such as less than about 16 wt%.

The humectants, such as glycerin and/or sorbitol, may be present in total amounts ranging from about 20 wt% to about 50 wt%, such as about 25 wt% to about 45 wt%, such as about 30 wt% to about 35 wt%. In one embodiment, the humectant of the present oral care composition is sorbitol, present in an amount of about 35 wt%.

In some embodiments, the orally acceptable vehicle is substantially anhydrous. Suitable orally acceptable humectants for such embodiments include but are not limited to polyethylene glycol, such as PEG400, PEG600, PEG/PPG copolymers, such as PEG/PPG 38/8 copolymer, PEG/PPG-1 16/66 copolymer sold as PLURACARE® L4370 and PLURACARE® L1220 from BASF corporation of Wyandotte, Michigan, respectively. In some embodiments, the humectant is present in a total amount of from about 2 wt% to about 55 wt% and typically from about 2 wt% to about 35 wt%. Propylene glycol or glycerin may also be present in an amount from about 0 wt% to about 15 wt%.

In some embodiments, the orally acceptable vehicle is a high water content orally acceptable vehicle and may include any known ingredients or additives. For example, the orally acceptable vehicle may include liquid mixtures of water, humectants such as glycerin and/or sorbitol and sodium chloride. In such embodiments, water is present typically in amount of at least about 21 wt% by weight, such as about 25 wt% to about 50 wt%, such as about 40 wt% to about 75 wt%, such as ranging from about 77 wt% to about 88 wt% by weight of the present oral care composition.

### Other Oral Care Ingredients

### Thickening agents

In some embodiments, the present oral care composition comprises a thickening agent. Any orally acceptable thickening agent can be used, including without limitation carbomers, also known as carboxyvinyl polymers, carrageenans, also known as Irish moss and more particularly -carrageenan (iota-carrageenan), cellulosic polymers such as hydroxyethylcellulose, carboxymethylcellulose (CMC) and salts thereof, *e.g.,* CMC sodium, natural gums such as low acyl gellan, xanthan, karaya gum arabic and tragacanth, colloidal magnesium aluminum silicate, and colloidal and/or thickener silica and mixtures of the same.

In certain embodiments, the thickener silica is a synthetic amorphous precipitated material of high surface area and internal pore volume to provide water absorption of about 50 ml or greater / 20 grams of silica and oil absorption of about 200 ml or greater /100g silica (per ASTM D281 method). Examples of thickener silicas which may be used are Zeodent® 165, Zeodent® 163 and Zeodent® 153 (from Huber); Aerosil® 200 and Sident® 22S (from Evonik); Sylodent® 15 and Perkasil® SM 660 (from W.R. Grace & Co.); MFIL®, MFIL®-P (From Madhu Silica, India) and Tixocil 43B (From Rhodia).

In some embodiments, the one or more thickening agents are present in a total amount of about 0.01 wt% to about 90 wt%. More typically, the one or more thickening agents are present in a total amount of about 0.25 wt% to about 4.0 wt%, such as about 2.6 wt%.

In some embodiments, particularly when the oral care composition is a dentifrice, the thickening agents include carrageenan and a thickener silica. The carrageenan may be present in amounts ranging from 0.05 wt% to 2.5 wt%, such as about 1.0 wt% to about 2 wt%, such as about 0.85 wt%. The thickener silica is typically present in amounts ranging from about 0.05 wt% to about 2.5 wt %, such as about 1.0 wt%-2 wt%, such as about 1.75 wt%.

### Structurants

In some embodiments, the present oral care composition includes structurants, such as for use in a structured mouth rinse, *i.e.* a mouth rinse that includes a material that provides rheological and structuring benefits. Such structurants include but are not limited to polymers or gum structurants that may swell or expand when hydrated to form random dispersion of independent microgel particles. Examples of polymers and gums structurants include: gellan gum, pectin, alginate, arabinogalactan, carrageenan, xanthum gum, guar gum, rhamsan gum, furcellaran gum, carboxymethylcellulose and cellulose. *See also* PCT Publication No. WO 2014088534. In some embodiments, sodium silicate may be used as a structurant.

In some embodiments, the viscosity of the oral care composition ranges from 200 cps to 1,000,000 cps. For example, the viscosity of a dentifrice from about 60,000 cps to 1,000,000 cps.

### Salts

In certain embodiments, particularly mouth rinse embodiments, the orally acceptable vehicle comprises 0.10 wt% to 1.0 wt% monovalent or divalent chloride salt such as CaCl₂, NaCl or a mixture thereof, to attain a viscosity ranging from 200 to 1000 cps, for example.

### Surfactants

In some embodiments the present oral care composition include surfactants that may be added to enhance stability of the formulation, help clean the oral cavity surfaces through detergency, and provide foam upon agitation, *e.g.,* during brushing or rinsing with an oral care composition of the present disclosure. Surface active agents may achieve increased prophylactic action, by thoroughly dispersing an anti-bacterial agent, for example, throughout the oral cavity. In various embodiments, suitable surface active agents may function as a surface active agent, emulsifier, and/or foam modulator.

Any orally acceptable surfactant, most of which are anionic, nonionic or amphoteric, can be used. Suitable anionic surfactants include without limitation water-soluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate (SLS), sodium cocoyl monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers such as polyoxomer 407, polyoxyethylene sorbitan esters such as polysorbate 20, fatty alcohol ethoxylates, alkylphenol ethoxylates, tertiary amine oxides, tertiary phosphine oxides, dialkyl sulfoxides and the like. Suitable amphoteric surfactants include without limitation derivatives of C₈₋₂₀ aliphatic secondary and tertiary amines having an anionic group such as carboxylate, sulfate, sulfonate, phosphate or phosphonate. A suitable example is cocoamidopropyl betaine. Typically, sodium lauryl sulfate is used as a surfactant in the present oral care compositions.

In some embodiments, one or more surfactants may be present in a total amount of from about 1.8 wt% to about 4 wt%. In some embodiments, one or more surfactants may be present in a total amount of from about 1.9 wt% to about 3 wt%. In some embodiments, one or more surfactants may be present in a total amount of about 2.5 wt%.

### Flavoring agents

Useful flavoring agents, which may be used with oral compositions of the present disclosure, include any material or mixture of materials operable to enhance the taste of the composition. Any orally acceptable natural or synthetic flavoring agent can be used, such as flavoring oils, flavoring aldehydes, esters, alcohols, similar materials, and combinations thereof. Flavoring agents include vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants, and mixtures thereof. Food-grade phosphoric acid may be used to acidify the oral care compositions of the present disclosure and provide a tangy and sour taste. Also encompassed within flavoring agents herein are ingredients that provide fragrance and/or other sensory effect in the mouth, including cooling or warming effects. Such ingredients include menthol, menthyl acetate, menthyl lactate, camphor, eucalyptus oil, eucalyptol, anethole, eugenol, cassia, oxanone, x-irisone, propenyl guaiethol, thymol, linalool, benzaldehyde, cinnamaldehyde, N-ethyl-p-menthan-3-carboxamine, N,2,3-trimethyl-2-isopropylbutanamide, 3-1-menthoxypropane-1,2-diol, cinnamaldehyde glycerol acetal (CGA), methone glycerol acetal (MGA) and mixtures thereof.

In some embodiments, one or more flavoring agents are optionally present in a total amount of about 0.01 wt% to about 5 wt%. In some embodiments, one or more flavoring agents are optionally present in a total amount of about 0.05 wt% to about 3 wt%. In some embodiments, one or more flavoring agents are optionally present in a total amount of about 0.1 wt% to about 2.5 wt%. In some embodiments, one or more flavoring agents are optionally present in a total amount from about 0.1 wt% to about 1.5 wt%, such as about 0.95 wt%.

### Sweeteners

Sweeteners among those useful herein include orally acceptable natural or artificial, nutritive or non-nutritive sweeteners. Such sweeteners include dextrose, polydextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, sucralose, dipeptide-based intense sweeteners, cyclamates, dihydrochalcones and mixtures thereof. Some embodiments optionally comprise one or more sweeteners. In some embodiments, the one or more optional sweeteners are present in a total amount from about 0.005% to about 5% w/w. In some embodiments, the one or more optional sweeteners are present in a total amount from about 0.01 to about 1 % w/w. In some embodiments, about 0.3% of sodium saccharin is used as a sweetener.

### Colorants

In some embodiments, a colorant may be included in the oral care compositions of the present disclosure. As used herein, the term "colorant" refers to a substance in the form of a dry powder or liquid that imparts color to another substance. Generally, colorants include dyes, lakes, pigments or combinations thereof. In some embodiments, mica, mica-based pearlescent pigments, talc, calcium carbonate and silica may also be used as colorants. More typically, the colorant is a dye, lake or a combination of one or more dyes and/or one or more lakes. Even more typically, pigments are incorporated into the oral care compositions of the present disclosure. Such pigments include non-toxic, water insoluble inorganic pigments such as titanium dioxide and chromium oxide greens, ultramarine blues and pinks and ferric oxides, *e.g.,* annatto extract, chlorophyllin-copper complex, canthaxanthin, β-carotene, synthetic iron oxide, TiO₂ and mixtures thereof.

The desired amount of colorant, such as a pigment, in some embodiments, is the amount of colorant which results in a final concentration of up to 12% colorant in the present oral care compositions, for example, about 0.1% to about 12%, about 0.1% to about 10%, about 0.1% to about 2% or about 0.1% to about 1% by weight. Typically, about 1 wt% titanium oxide is used in the present oral care compositions.

### Antioxidants

In some embodiments, antioxidants are included in the present oral care compositions. Suitable antioxidants include ascorbic acid, ascorbyl palmitate, thiodipropionic acid, calcium ascorbate, dilauryldithiopropionate, gum guaiac, sodium ascorbate, butylated hydroxyl toluene, butylated hydroxyl anisole, and tocopherols. Mixtures of antioxidants can also be used.

When present, the antioxidant is added in a level effective to reduce or mitigate discoloration that may otherwise result from oxidation of the components of the toothpastes. When antioxidants are present, typical levels range from about 0.01 wt% to about 1 wt%.

### Preservatives

Suitable preservatives such as benzyl alcohol and may also be included in the present oral care compositions. Other preservatives which may be used include methyl, ethyl, butyl, propyl and isopropyl esters of parahydroxybenzoic acid. Other suitable preservatives include formaldehyde, dimethyl hydantoin and sodium silicate. In some embodiments, the present oral care compositions includes from about 0 wt% to about 0.8 wt%, and typically from about 0.05 wt% to about 0.5 wt%, more typically about 0.1 wt% to 0.3 wt% preservative. Typically, benzyl alcohol is used in the present oral care compositions.

### Methods

The present disclosure also provides methods of making the oral care compositions of the present disclosure. The methods include combining ingredients including a calcium-based abrasive, such as precipitated calcium carbonate, a polyethylene glycol such as PEG 6000 and an anti-bacterial agent, such as triclosan, with an orally acceptable vehicle. In some embodiments, buffering agents and/or pH adjusting agents are included to obtain a pH of the present oral care composition as disclosed herein, for example, a pH less than 9.0, such as 8.68. In other embodiments, a buffering agent such as sodium silicate is omitted to obtain a desired pH, *e.g.* less than 9.0, such as 8.68. In various embodiments, additional active ingredients including fluoride, bicarbonate, anti-calculus agents, whitening agents and stannous ions as disclosed herein may also be included in the present oral care compositions. Additional oral care ingredients may also be included, such as thickening agents, surfactants, sweeteners, flavors, preservatives and colorants.

In some embodiments, the time to prepare the oral care composition ranges from 1 hour to 4 hours, such as from about 1 hour to about 3 hours, such as from about 1 hour to about 2 hours. Typically, the time for preparing an oral care composition of the present disclosure is about 1.5 hours or less.

In some embodiments, the present oral care composition is prepared by mixing a humectant, such as sorbitol, with water at temperatures ranging from 60°C to 62°C to form an orally acceptable vehicle. Typically, the water is pasteurized, filtered and purified water heated to 85°C.

Typically, one or more thickening agents, such as carrageenan and/or a thickening silica, is added to the orally acceptable vehicle. In some embodiments, active ingredients, such as sodium monoflurophosphate and sodium bicarbonate are added to the thickening agent, such as carrageenan, before the thickening agent is combined with the orally acceptable vehicle. A sweetener may also be added to the thickening agent, such as carrageenan, and the optional active ingredients. The thickening agent and optional additional ingredients are mixed under heat at temperatures ranging from 55°C-65°C, such as 55°C to 60°C or such as 64°C to 65°C before combining with the orally acceptable vehicle.

In some embodiments, a buffering agent, such as sodium silicate, may then be added to the above-described mixture.

In some embodiments, a calcium-based abrasive, such as precipitated calcium carbonate, is then added to the orally acceptable vehicle and the additional ingredients, *e.g.,* thickening agents, sodium monfluorophospate, sodium bicarbonate and a sweetener. The precipitated calcium carbonate may be added under a closed vacuum and simultaneously de-aerated to produce a smooth air free paste. In various embodiments, a colorant, such as titanium dioxide is added at the same time as the calcium based abrasive.

In some embodiments, a surfactant, such as sodium lauryl sulfate may be added to the above-described mixture containing the calcium-based abrasive. Typically, the surfactant is in the form of granules and is slowly added under open vacuum while stirring at temperatures ranging from 52° C to 46° C.

In some embodiments, a PEG-6000 premix is prepared by combining PEG-6000 with the halogenated diphenyl ether anti-bacterial agent, *e.g.,* triclosan. In other embodiments, a preservative, such as benzyl alcohol and a flavoring are first mixed with the PEG-6000 until the PEG-6000 is dissolved, followed by the addition of the anti-bacterial agent. The PEG-6000 premix is then added to the above described mixture containing the calcium-based abrasive and, optionally, the surfactant to form an oral care composition of the present disclosure.

Methods are also provided herein to promote oral health in a human or animal subject comprising contacting an oral surface with an oral care composition of the present disclosure, which includes a calcium-based abrasive, a polyethylene glycol as described above, an halogenated diphenyl ether anti-bacterial agent, an orally acceptable vehicle and other optional ingredients at a suitable pH as disclosed herein. As used herein, the phrase "promote oral health" encompasses preventing, curing, reversing, attenuating, alleviating, ameliorating, minimizing, suppressing or halting the deleterious effects of one or more oral conditions in a human or animal. The phrase "oral conditions" include but are not limited to plaque, tartar, stains, halitosis, gingivitis, periodontitis, and combinations thereof.

As used herein "animal subject" includes non-human mammals such as canines, felines, and horses. The present oral care composition is contacted with an oral surface of the mammalian subject to thereby promote oral health in a highly efficacious manner.

In various embodiments, the present oral care compositions prepared in accordance with the present disclosure may be applied regularly to an oral surface, for example on a daily basis, at least one time daily for multiple days, or alternately every second or third day. In some embodiments, the present oral care composition is applied to the oral surfaces from 1 to 3 times daily, for at least 2 weeks up to 8 weeks, from four months to three years, or more up to lifetime.

The examples and other embodiments described herein are exemplary and not intended to be limiting in describing the full scope of compositions and methods of this disclosure. Equivalent changes, modifications and variations of specific embodiments, materials, compositions and methods may be made within the scope of the present disclosure, with substantially similar results.

### EXAMPLES

### Example 1-Dentifrice Formulations

Seven dentifrice formulations A, C, D, E, F, G and H were prepared as described below by mixing the ingredients in the amounts specified in Table 1. A competitor dentifrice formulation comprising VEEGUM® was designated as Formula B (Table 2).

A stainless steel vessel (SS vessel) equipped with a stirrer was charged with 70% sorbitol and heated to 60°C while stirring. Pasteurized, filtered, purified water was added to the sorbitol and heated to 85°C.

A premix containing carrageenan, sodium monoflurophospate, sodium saccharin and sodium bicarbonate was slowly added to a separate vessel (SS vessel) to form a gel mass. The gel mass was mixed until no lumps were observed (about 20 minutes) at a temperature ranging from 64°C to 65°C. 0.5 wt% pasteurized water was added to the SS vessel to compensate for water loss. The gel mass was then transferred to the stainless steel mixing vessel (Alpro Mixer).

Liquid sodium silicate (Formulas A, C, E, F and H) was then added to the stainless steel mixing vessel (Alpro Mixer). The mixture was stirred for 2 minutes.

The stainless steel mixing vessel was then charged with thickening silica (ZEODENT® 165/MFIL®-P and mixed until completely dispersed in the sorbitol, water, gel mass and liquid sodium silicate mixture described above (about 7 minutes).

Two separate lots of precipitated calcium carbonate and titanium dioxide were then added to the above-described mixture. The mixture was stirred under closed vacuum (650 mm Hg) and simultaneously de-aerated until the temperature reached 52°C. Full vacuum (720 mm Hg) was then applied and the mixture de-aerated. This process was completed in about 15 minutes.

Sodium lauryl sulfate granules were slowly added to the above-described mixture at 52°C while stirring under open vacuum (650 mm Hg) until the temperature dropped to 46 °C (about 10 minutes). The mixture was stirred for 15 minutes. The vacuum was monitored to ensure the pressure did not fall below 600 mm Hg. Addition of sodium lauryl sulfate granules was discontinued when the pressure dropped below 600 mm Hg, at which point full vacuum (720 mm Hg) was applied and the sodium lauryl sulfate addition was then continued under open vacuum.

PEG-6000 (as a fine powder) was combined with a flavoring agent and benzyl alcohol and stirred until the PEG-6000 was dissolved. Triclosan was then added and dissolved to form a PEG-6000 premix. The PEG-6000 premix solution was slightly hazy. Without being bound by theory, it is believed that the haziness may be due to a complete dispersion of solids caused by emulsification between the PEG-6000 and benzyl alcohol. The above-described PEG-6000 premix was added to the stainless steel mixing vessel and combined with the previously added ingredients under closed vacuum (650 mm Hg) until the premix was completely dispersed. The mixture was then subjected to full vacuum (720 mm Hg), de-aerated and stirred for five minutes.

After completion of the above-described processes, the mixer was stopped and the vacuum released. The batch was checked for graininess. Any batch found to be grainy was subjected to full vacuum (720 mm Hg) and mixed for five minutes. Samples of the batches were drawn and checked for taste, color, odor, texture, pH, specific gravity and dissolution of the sodium lauryl sulfate granules.

All of the formulas A, C, D, E, F, G and H, prepared as described above, contained 39% calcium carbonate and 0.3% triclosan as specified in Table 1; however, only formulas E, F and G contained PEG 6000. Formulas A (control), E, F and H each contained 1% sodium silicate. Formula C contained 3% sodium silicate. Formulas D and G did not contain sodium silicate. Due to the lack of sodium silicate, Formulas D and G exhibited lower pH values than Formulas A, C, E, F and H, *i.e.,* 8.9 and 8.68, respectively. *See* Table 1.

**Table 1. Calcium Carbonate Based Formulation Evaluated for Triclosan Uptake and Retention**

| | (wt%) | (wt%) | (wt%) | (wt%) | (wt%) | (wt%) | (wt%) |
|---|---|---|---|---|---|---|---|
| **Changes** | **Chalk TP Contr ol** | **Chalk TP 3% sodium silicate** | **Chalk TP no sodium silicate** | **Chalk TP PEG 6000 (0.3%)** | **Chalk TP PEG 6000 (0.1%)** | **Chalk TP PEG 6000 (0.3%), Silicate** (0%) | **Chalk TP PEG 600(0.3%)** |
| **Formula** | **A** | **C** | **D** | **E** | **F** | **G** | **H** |
| Sorbitol, water and minors (color, flavor, sweetener) | 53.07 | 51.07 | 54.07 | 52.77 | 52.97 | 53.77 | 52.77 |
| Thickening agents | 2.57 | 2.57 | 2.57 | 2.57 | 2.57 | 2.57 | 2.57 |
| Sodium Silicate | 1 | 3 | 0 | 1 | 1 | 0 | 1 |
| Sodium bicarbonate | 0.5 | 0.5 | 0.5 | 0.5 | 0.5 | 0.5 | 0.5 |
| Sodium Monofluorophosphate | 0.76 | 0.76 | 0.76 | 0.76 | 0.76 | 0.76 | 0.76 |
| Precipitated Calcium Carbonate | 39 | 39 | 39 | 39 | 39 | 39 | 39 |
| triclosan | 0.3 | 0.3 | 0.3 | 0.3 | 0.3 | 0.3 | 0.3 |
| Sodium Lauryl Sulfate (surfactant) | 2.5 | 2.5 | 2.5 | 2.5 | 2.5 | 2.5 | 2.5 |
| Benzyl Alcohol (preservatives) | 0.3 | 0.3 | 0.3 | 0.3 | 0.3 | 0.3 | 0.3 |
| PEG 6000 | 0 | 0 | 0 | 0.3 | 0.1 | 0.3 | 0 |
| PEG 600 | 0 | 0 | 0 | 0 | 0 | 0 | 0.3 |
| Total | 100 | 100 | 100 | 100 | 100 | 100 | 100 |
| pH | 9.37 | 9.54 | 8.90 | 9.20 | 9.25 | 8.68 | 9.21 |

### Example 2. Uptake and Retention

The effect of polyethylene glycol, such as PEG 6000, in the present oral care compositions on uptake and retention of triclosan to tooth surfaces was assessed using disks of saliva-coated hydroxyapatite (SCHAP), the mineral phase of dental enamel, as an *in vitro* experimental model for human teeth. The *in vitro* assessment has been found to be correlated with *in vivo* delivery of and retention of anti-bacterial agents on oral surfaces.

For the test of uptake of triclosan to a SCHAP disk, parafilm stimulated whole saliva was collected into a beaker and clarified by centrifugation at 10,000 rpm for 10 minutes. Each hydroxyapatite disk was hydrated with sterile water in a test tube. The water was then removed and replaced with 2 milliliters of the clarified saliva. A salivary pellicle was formed by incubating the disk overnight at 37° C with continuous shaking in a water bath. After this treatment, excess saliva was removed by aspiration, the disks were transferred to a new tube and treated with dentifrice slurry. The dentifrice slurry was prepared by diluting 5 grams of each of the dentifrice formulas A-G as described herein with 10 grams of water followed by stirring in a beaker with a stir bar. Water was also used as a control dentifrice formulation.

The disks were then incubated with 1 milliliter of dentifrice slurry at 37° C. After 30 minutes, the supernatant was aspirated from each disk, the disks were each then transferred into a new tube and 5 milliliters of water was added to each new tube followed by shaking the disk with a Vortex for five seconds. The disks were then each transferred to a new tube and the washing procedure repeated three times in total. To assess triclosan uptake, 1.0 ml of ethanol was added to each disk and left at room temperature for 1 hour to extract the absorbed triclosan into the ethanol and shaken with a Vortex for ten seconds. The ethanol was aspirated and transferred into HPLC (high performance liquid chromatography) vials for determination of the concentration of triclosan present.

To assess triclosan retention, the procedure was the same as described above for triclosan uptake, except that after the disks were washed three times, each disk was placed in 1 milliliter of saliva and incubated at 37°C for four hours. As described above for triclosan uptake, 1.0 ml of ethanol was then added to each disk and left at room temperature for 1 hour to extract the absorbed triclosan into the ethanol and shaken with a Vortex for ten seconds. The ethanol was then aspirated and transferred into HPLC (high performance liquid chromatography) vials for determination of the concentration of triclosan retained on the disks.

### Example 3. Results

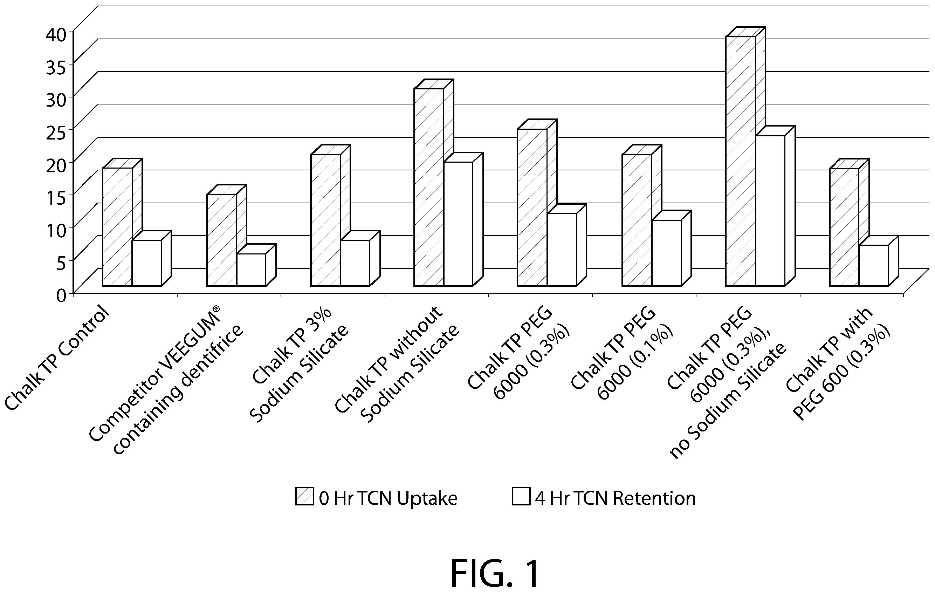

As evidenced in Table 2, high molecular weight polyethylene glycol, such as PEG 6000, surprisingly improves the uptake (0 hours) and retention (4 hours) of an anti-bacterial such as triclosan. As shown in Table 2, formulations containing PEG 6000 have better uptake at 0 hours on hydroxyapatite (HAP) disks than control Formula A. For example, as seen in Table 2 the PEG-6000 containing formulations resulted in 24 µg (Formula E), 20 µg (Formula F) and 38 µg (Formula G) of triclosan on hydroxyapatite disks at 0 hours in comparison to control Formula A, *i.e.* 18 µg of triclosan/HAP at 0 hours. *See also* FIG. 1.

Furthermore, Table 2 also shows that formulations containing PEG 6000 have better retention at 4 hours on hydroxyapatite disks than control Formula A. For example, as seen in Table 2 the PEG-6000 containing formulations resulted in 11 µg (Formula E), 10 µg (Formula F) and 23 µg (Formula G) of triclosan on hydroxyapatite disks at 4 hours in comparison to control Formula A, *i.e.* 7 µg of triclosan/HAP at 4 hours. *See also* FIG. 1.

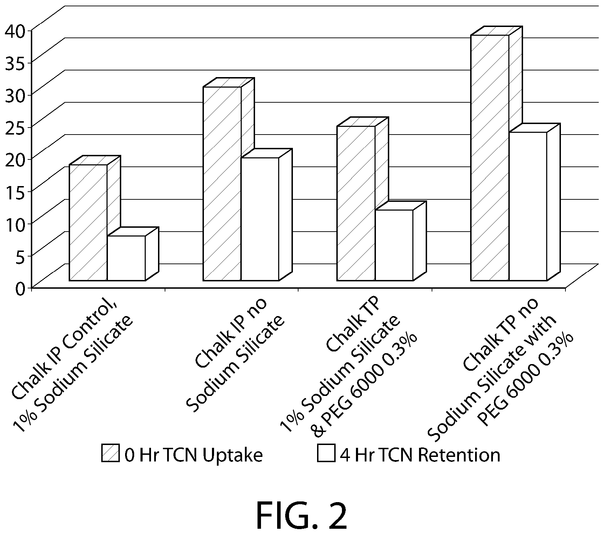

Surprisingly, omission of sodium silicate, which results in a slightly lower pH, also unexpectedly improved the uptake of triclosan. For example, as seen in Table 2 the formulations without sodium silicate resulted in 30 µg (Formula D, pH 8.9) and 38 µg (Formula G, pH 8.68) of triclosan on hydroxyapatite disks at 0 hours in comparison to control Formula A, pH 9.37, *i.e.* 18 µg of triclosan/HAP at 0 hours. *See also* FIG. 1 and FIG. 2.

Furthermore, omission of sodium silicate and the resulting lower pH also unexpectedly improved the retention of triclosan. For example, as seen in Table 2 the formulations without sodium silicate resulted in 19 µg (Formula D) and 23 µg (Formula G) of triclosan on hydroxyapatite disks at 4 hours in comparison to control Formula A, *i.e.* only 7 µg of triclosan/HAP at 4 hours. *See also* FIG. 1 and FIG. 2.

Moreover, Table 2 shows that combining PEG 6000 with a lower pH (no sodium silicate) synergistically (greater than additive effect) increased the uptake and retention of triclosan. *See* for example, Formula G, which contains 0.3% PEG 6000 and has a pH of 8.68 (no sodium silicate), retained 38 µg triclosan/HAP at 0 hours and 23 µg triclosan/HAP after 4 hours in comparison to control Formula A, *i.e.,* 18 µg of triclosan/HAP at 0 hours and 7 µg triclosan/HAP at 4 hours. See also FIG. 1 and FIG. 2.

In view of the foregoing, the present Examples demonstrate the superior uptake and retention of an anti-bacterial such as triclosan using the oral care compositions of the present disclosure.

**Table 2. Triclosan Uptake and Retention Data**

| | | | Triclosan retention in µg/HAP | |
|---|---|---|---|---|
| Formula | Product | pH | 0 Hours | 4 Hours |
| A | CHALK TP Control | 9.37 | 18 | 7 |
| B | Competitor VEEGUM® containing dentifrice* | 9.28 | 14 | 5 |
| C | CHALK TP with 3% sodium silicate | 9.54 | 20 | 7 |
| D | CHALK TP without sodium silicate | 8.90 | 30 | 19 |
| E | CHALK TP with PEG 6000 (0.3%) with sodium silicate | 9.20 | 24 | 11 |
| F | CHALK TP with PEG 6000 (0.1%) with sodium silicate | 9.25 | 20 | 10 |
| G | CHALK TP with PEG 6000 (0.3%) without sodium silicate | 8.68 | 38 | 23 |
| H | CHALK TP with PEG 600 (0.3%) with sodium silicate | 9.21 | 18 | 6 |

| | | | | |
|---|---|---|---|---|
| *Competitor sample containing Triclosan | | | | |

## Claims

1. An oral care composition comprising:
(i) a calcium-based abrasive:
(ii) a polyethylene glycol having an average molecular weight ranging from 1500 to 6000 daltons in an amount of 0.1 wt% to 0.3 wt%;
(iii) an anti-bacterial agent; and
(iv) an orally acceptable vehicle,
wherein the anti-bacterial agent is a halogenated diphenyl ether.

2. The oral care composition of claim 1, wherein the anti -bacterial agent is 5-chloro- 2-(2,4-dichlorophenoxy)phenol (triclosan).

3. The oral care composition of claim 1 or 2, wherein the polyethylene glycol has an average molecular weight of 6000 daltons (PEG 6000).

4. The oral care composition of any one of the preceding claims, wherein the calcium-based abrasive is selected from the group consisting of dicalcium orthophosphate dihydrate, calcium pyrophosphate, n-calcium pyrophosphate, tricalcium phosphate, calcium polymetaphosphate, precipitated calcium carbonate, natural calcium carbonate and combinations thereof.

5. The oral care composition of claim 4, wherein the calcium-based abrasive is precipitated calcium carbonate.

6. The oral care composition of any one of the preceding claims, wherein a pH of the oral care composition is less than about 9.0, optionally wherein a pH of the oral care composition ranges from 8.6 to 8.9.

7. The oral care composition of any one of the preceding claims, wherein the oral care composition does not contain sodium silicate.

8. The oral care composition of any one of the preceding claims, wherein the oral care composition further comprises a carrageenan and a thickener silica.

9. The oral care composition of claim 8, wherein the carrageenan is present in an amount ranging from 0.5wt% to 1.0 wt% and the thickener silica is present in an amount ranging from 1% to 2%.

10. The oral care composition of any one of the preceding claims, wherein the oral care composition is a dentifrice or a mouth rinse.

11. The oral care composition of any one of the preceding claims, wherein the oral care composition does not include a copolymer of monoalkyl esters of poly (methyl vinyl ether/maleic acid).

12. A method of preparing an oral care composition according to anyone of claims 1 to 11, comprising:
a) combining a humectant and water to form an orally acceptable vehicle:
b) adding a calcium-based abrasive to the orally acceptable vehicle;
c) combining a polyethylene glycol having an average molecular weight ranging from 1500 to 6000 daltons, an anti-bacterial agent, and, optionally, an ingredient selected from the group consisting of a flavoring agent and a preservative to form a premix;
d) adding the premix of step c) to the orally acceptable vehicle containing the calcium base abrasive of step b) to form the oral care composition,
wherein the anti-bacterial agent is a halogenated diphenyl ether.

13. The method of claim 12, wherein the method further comprises adding at least one thickening agent to the orally acceptable vehicle of step a) before adding the calcium-based abrasive to the orally acceptable vehicle of step b), optionally wherein the at least one thickening agent comprises a thickening agent selected from the group consisting of carrageenan, a thickening silica and a combination thereof.

14. The method of claim 12 or 13, wherein the polyethylene glycol has an average molecular weight of 6000 daltons and wherein the anti -bacterial agent is 5-chloro-2-(2,4-dichlorophenoxy)phenol (triclosan).

## Patentansprüche

1. Mundpflegezusammensetzung, umfassend:
(i) ein Calcium basiertes Abrasivmittel:
(ii) ein Polyethylenglykol mit einem durchschnittlichen Molekulargewicht im Bereich von 1500 bis 6000 Dalton in einer Menge von 0,1 bis 0,3 Gew.-%;
(iii) ein antibakterielles Mittel; und
(iv) ein oral annehmbares Vehikel,
wobei das antibakterielle Mittel ein halogenierter Diphenylether ist.

2. Mundpflegezusammensetzung nach Anspruch 1, wobei das antibakterielle Mittel 5-Chlor-2-(2,4-dichlorphenoxy)phenol (Triclosan) ist.

3. Mundpflegezusammensetzung nach Anspruch 1 oder 2, wobei das Polyethylenglykol ein durchschnittliches Molekulargewicht von 6000 Dalton (PEG 6000) hat.

4. Mundpflegezusammensetzung nach irgendeinem der vorhergehenden Ansprüche, wobei das Calcium basierte Abrasivmittel ausgewählt ist aus der Gruppe bestehend aus Dicalciumorthophosphat-Dihydrat, Calciumpyrophosphat, n-Calciumpyrophosphat, Tricalciumphosphat, Calciumpolymetaphosphat, ausgefälltes Calciumcarbonat, natürliches Calciumcarbonat und Kombinationen davon.

5. Mundpflegezusammensetzung nach Anspruch 4, wobei das Calcium basierte Abrasivmittel ausgefälltes Calciumcarbonat ist.

6. Mundpflegezusammensetzung nach irgendeinem der vorhergehenden Ansprüche, wobei ein pH-Wert der Mundpflegezusammensetzung weniger als etwa 9,0 ist, gegebenenfalls ist der pH-Wert der Mundpflegezusammensetzung in einem Bereich von 8,6 bis 8,9.

7. Mundpflegezusammensetzung nach irgendeinem der vorhergehenden Ansprüche, wobei die Mundpflegezusammensetzung kein Natriumsilikat enthält.

8. Mundpflegezusammensetzung nach irgendeinem der vorhergehenden Ansprüche, wobei die Mundpflegezusammensetzung weiterhin ein Karrageenan und ein Verdickersilka umfasst.

9. Mundpflegezusammensetzung nach Anspruch 8, wobei das Karrageenan in einer Menge im Bereich von 0,5 bis 1,0 Gew.-% und das Verdickersilka in einer Menge im einem Bereich von 1 % bis 2 % vorliegt.

10. Mundpflegezusammensetzung nach irgendeinem der vorhergehenden Ansprüche, wobei die Mundpflegezusammensetzung ein Zahnputzmittel oder eine Mundspülung ist.

11. Mundpflegezusammensetzung nach irgendeinem der vorhergehenden Ansprüche, wobei die Mundpflegezusammensetzung kein Copolymer eines Monoalkylesters von Poly(methylvinylether/Maleinsäure) einschließt.

12. Verfahren zum Herstellen einer Mundpflegezusammensetzung nach irgendeinem der Ansprüche 1 bis 11, umfassend:
a) das Kombinieren eines Befeuchtungmittels und Wasser, um ein oral annehmbares Vehikel zu bilden;
b) Zugabe eines Calcium basierten Abrasivmittels zu dem oral annehmbaren Vehikel;
c) Kombinieren eines Polyethylenglykols mit einem durchschnittlichen Molekulargewicht im Bereich von 1500 bis 6000 Daltons, einem antibakteriellen Mittel und gegebenenfalls einen Bestandteil, ausgewählt aus der Gruppe bestehend aus einem Geschmacksmittel und einem Konservierungsmittel, um eine Vormischung zu bilden;
d) Zugabe der Vormischung aus Schritt c) zu dem oral annehmbaren Vehikel, der das Calcium basierte Abrasivmittel nach Schritt b) enthält, um die Mundpflegezusammensetzung zu bilden,
wobei das antibakterielle Mittel ein halogenierter Diphenylether ist.

13. Verfahren nach Anspruch 12, wobei das Verfahren weiterhin umfasst die Zugabe von mindestens einem Verdickungsmittel zu dem oral annehmbaren Vehikel von Schritt a) vor Zugabe des Calcium basierten Abrasivmittels zu dem oral annehmbaren Vehikel von Schritt b), wobei gegebenenfalls das mindestens eine Verdickungsmittel ein Verdickungsmittel umfasst ausgewählt aus der Gruppe bestehend aus Karrageenan, einem Verdickersilika und einer Kombination davon.

14. Verfahren nach Anspruch 12 oder 13, wobei das Polyethylenglykol ein durchschnittliches Molekulargewicht von 6000 Daltons hat und wobei das antibakterielle Mittel 5-Chlor-2-(2,4-dichlorphenoxy)phenol ist (Triclosan).

## Revendications

1. Composition de soin bucco-dentaire comprenant :
(i) un abrasif à base de calcium :
(ii) un polyéthylène glycol ayant un poids moléculaire moyen allant de 1 500 à 6 000 daltons en une quantité de 0,1 % en poids à 0,3 % en poids ;
(iii) un agent antibactérien ; et
(iv) un véhicule acceptable par voie orale,
dans laquelle l'agent antibactérien est un diphényléther halogéné.

2. Composition de soin bucco-dentaire selon la revendication 1, dans laquelle l'agent anti-bactérien est le 5-chloro-2-(2,4-dichlorophénoxy)phénol(triclosan).

3. Composition de soin bucco-dentaire selon la revendication 1 ou 2, dans laquelle le polyéthylène glycol a un poids moléculaire moyen de 6 000 daltons (PEG 6000).

4. Composition de soin bucco-dentaire selon l'une quelconque des revendications précédentes, dans laquelle l'abrasif à base de calcium est choisi dans le groupe constitué par l'orthophosphate dicalcique dihydraté, le pyrophosphate de calcium, le pyrophosphate de n-calcium, le phosphate tricalcique, le polymétaphosphate de calcium, le carbonate de calcium précipité, le carbonate de calcium naturel et une combinaison de ceux-ci.

5. Composition de soin bucco-dentaire selon la revendication 4, dans laquelle l'abrasif à base de calcium est du carbonate de calcium précipité.

6. Composition de soin bucco-dentaire selon l'une quelconque des revendications précédentes, dans laquelle un pH de la composition de soin bucco-dentaire est inférieur à environ 9,0, éventuellement dans laquelle un pH de la composition de soin bucco-dentaire est compris entre 8,6 et 8,9.

7. Composition de soin bucco-dentaire selon l'une quelconque des revendications précédentes, dans laquelle la composition de soin bucco-dentaire ne contient pas de silicate de sodium.

8. Composition de soin bucco-dentaire selon l'une quelconque des revendications précédentes, dans laquelle la composition de soin bucco-dentaire comprend en outre un carraghénane et une silice épaississante.

9. Composition de soin bucco-dentaire selon la revendication 8, dans laquelle le carraghénane est présent en une quantité allant de 0,5 % en poids à 1,0 % en poids et la silice épaississante est présente en une quantité allant de 1 % à 2 %.

10. Composition de soin bucco-dentaire selon l'une quelconque des revendications précédentes, dans laquelle la composition de soin bucco-dentaire est un dentifrice ou un rince-bouche.

11. Composition de soin bucco-dentaire selon l'une quelconque des revendications précédentes, dans laquelle la composition de soin bucco-dentaire ne comprend pas de copolymère d'esters monoalkyliques de poly(éther méthylvinylique/acide maléique).

12. Procédé de préparation d'une composition de soin bucco-dentaire selon l'une quelconque des revendications 1 à 11, comprenant :
a) la combinaison d'un humectant et d'eau pour former un véhicule acceptable par voie orale :
b) l'ajout d'un abrasif à base de calcium au véhicule acceptable par voie orale ;
c) la combinaison d'un polyéthylène glycol ayant un poids moléculaire moyen allant de 1 500 à 6 000 daltons, d'un agent antibactérien, et, éventuellement, d'un ingrédient choisi dans le groupe constitué par un agent aromatisant et un conservateur pour former un prémélange ;
d) l'ajout du prémélange de l'étape c) au véhicule acceptable par voie orale contenant l'abrasif à base de calcium de l'étape b) pour former la composition de soin bucco-dentaire,
dans lequel l'agent antibactérien est un diphényléther halogéné.

13. Procédé selon la revendication 12, dans lequel le procédé comprend en outre l'ajout d'au moins un agent épaississant au véhicule acceptable par voie orale de l'étape a) avant l'ajout de l'abrasif à base de calcium au véhicule acceptable par voie orale de l'étape b), éventuellement dans lequel l'au moins un agent épaississant comprend un agent épaississant choisi dans le groupe constitué par un carraghénane, une silice épaississante et une combinaison de ceux-ci.

14. Procédé selon la revendication 12 ou 13, dans lequel le polyéthylène glycol a un poids moléculaire moyen de 6 000 daltons et dans lequel l'agent antibactérien est le 5-chloro-2-(2,4-dichlorophénoxy)phénol(triclosan).
